(19) Europäisches Patentamt
European Patent Office
Office européen des brevets

(11) **EP 2 150 179 B1**

(12) **EUROPÄISCHE PATENTSCHRIFT**

(45) Veröffentlichungstag und Bekanntmachung des Hinweises auf die Patenterteilung:
**24.04.2013 Patentblatt 2013/17**

(21) Anmeldenummer: **08759740.7**

(22) Anmeldetag: **19.05.2008**

(51) Int Cl.:
*A61B 6/03* $^{(2006.01)}$

(86) Internationale Anmeldenummer:
**PCT/EP2008/056115**

(87) Internationale Veröffentlichungsnummer:
**WO 2008/142048 (27.11.2008 Gazette 2008/48)**

(54) **AUSWAHLVERFAHREN FÜR ZWEI KONTRASTMITTEL ZUR VERWENDUNG IN EINER DUAL-ENERGY-CT-UNTERSUCHUNG, KONTRASTMITTELKOMBINATION UND ERZEUGUNG VON CT-AUFNAHMEN MIT EINER KONTRASTMITTELKOMBINATION MIT UNTERSCHIEDLICHEN ENERGIESPEKTREN**

SELECTION METHOD FOR TWO CONTRAST MEDIA FOR USE IN A DUAL-ENERGY CT EXAMINATION, CONTRAST MEDIA COMBINATION AND GENERATION OF CT IMAGES USING A CONTRAST MEDIA COMBINATION AND DIFFERENT ENERGY SPECTRA

PROCÉDÉ DE SÉLECTION DE DEUX AGENTS DE CONTRASTE DESTINÉS À ÊTRE UTILISÉS LORS D'UN EXAMEN PAR CT À DOUBLE ÉNERGIE, COMBINAISON D'AGENTS DE CONTRASTE ET PRODUCTION D'IMAGES DE CT À L'AIDE D'UNE COMBINAISON D'AGENTS DE CONTRASTE ET DE SPECTRES D'ÉNERGIE DIFFÉRENTS

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **24.05.2007 DE 102007024158**

(43) Veröffentlichungstag der Anmeldung:
**10.02.2010 Patentblatt 2010/06**

(73) Patentinhaber:
• **Siemens Aktiengesellschaft**
**80333 München (DE)**
• **Bayer Pharma Aktiengesellschaft**
**13353 Berlin (DE)**

(72) Erfinder:
• **KRAUSS, Bernhard**
**90518 Altdorf (DE)**
• **PIETSCH, Hubertus**
**14532 Kleinmachnow (DE)**

(74) Vertreter: **Maier, Daniel Oliver**
**Siemens AG**
**Postfach 22 16 34**
**80506 München (DE)**

(56) Entgegenhaltungen:
**US-A1- 2005 084 069**

• **JOHNSON THORSTEN R C ET AL: "Material differentiation by dual energy CT: initial experience." EUROPEAN RADIOLOGY ONLINE, Bd. 17, Nr. 6, Dezember 2006 (2006-12), Seiten 1510-1517, XP002497949 ISSN: 0938-7994**
• **CARDINAL H N ET AL: "Experimental and theoretical x-ray imaging performance comparison of iodine and lanthanide contrast agents.", MEDICAL PHYSICS 1993 JAN-FEB LNKD- PUBMED:8455493, vol. 20, no. 1, January 1993 (1993-01), pages 15-31, ISSN: 0094-2405**
• **ANONYMOUS: SPIE, PO BOX 10 BELLINGHAM WA 98227-0010, USA, XP040548853,**

**Beschreibung**

Auswahlverfahren für zwei Kontrastmittel zur Verwendung in einer Dual-Energy-CT-Untersuchung, Kontrastmittelkombination und Erzeugung von CT-Aufnahmen mit einer Kontrastmittelkombination mit unterschiedlichen Energiespektren

Die Erfindung betrifft ein Verfahren zur Auswahl zweier Kontrastmittel zur Verwendung in einer Dual-Energy-CT-Untersuchung eines Patienten. Weiterhin wird ein Verfahren zur Erzeugung von CT-Aufnahmen eines Patienten und eine Kombination zweier Kontrastmittel offenbart, vorgesehen zur Applikation bei einer CT-Untersuchung mit mindestens zwei unterschiedlichen Röntgenenergiespektren zur Beurteilung des Anteils einer ersten und einer zweiten Gewebeart, wobei zwischen der ersten und zweiten Gewebeart einem HU-Werte-Diagramm eine Verbindungslinie mit einer Grundsteigung gebildet wird. Außerdem wird auch eine Kontrastmittelkombination und die Erzeugung von CT-Aufnahmen mit dieser Kontrastmittelkombination unter Betrachtung zweier unterschiedlicher Energiespektren offenbart.

Verfahren zur Bestimmung einer Kontrastmittel-Konzentration im Körpermaterial eines menschlichen oder tierischen Patienten und zur gleichzeitigen Differenzierung zweier unterschiedlicher Gewebearten sind allgemein bekannt. Insbesondere werden hierbei die folgenden Verfahrensvarianten verwendet:

Zur Bestimmung einer Kontrastmittel-Konzentration mittels Computertomographie (CT) sind bisher zwei unterschiedliche Techniken bekannt. Bei der ersten Technik kann jeweils vor und nach einer Verabreichung von Kontrastmittel eine Computertomographie-Aufnahme des Körperbereiches aufgezeichnet werden, in dem die Kontrastmittel-Konzentration gemessen werden soll. Nach einer Registrierung der beiden dabei erhaltenen CT-Bilder werden diese voneinander subtrahiert, um die durch das Kontrastmittel verursachte Anhebung der Röntgenschwächungswerte für jeden Bildpunkt bzw. jedes Voxel zu erhalten. Diese Anhebung der Röntgenschwächungswerte ist proportional zur Konzentration des Kontrastmittels. Durch die hierbei erforderlichen Computertomographie-Aufnahmen zu unterschiedlichen Zeiten können jedoch Registrierungs- und/oder Bewegungsartefakte auftreten, die zu einer fehlerhaften Bestimmung führen können. Bei Einsatz eines Kontrastmittels, das sich nur langsam im Körpermaterial ansammelt, muss zudem eine unerwünscht lange Wartezeit zwischen den beiden Computertomographie-Aufnahmen eingehalten werden.

Die zweite bekannte Technik nutzt den Einsatz eines Mehr-Energie-Computertomographen zur gleichzeitigen Aufzeichnung von zwei Computertomographie-Aufnahmen mit unterschiedlicher spektraler Verteilung der Röntgenstrahlung, d.h. unterschiedlicher Röntgenenergie. In einer Variante dieser Technik werden zunächst die Bilddatensätze für beide Röntgenenergien getrennt voneinander rekonstruiert. Anschließend werden die gemessenen Röntgenschwächungswerte für jedes Voxel in die molekulare Dichte von zwei Basismaterialien zerlegt (2-Material-Zerlegung), von denen ein Basismaterial das Kontrastmittel darstellt. Aus den beiden aus der Zerlegung resultierenden Gleichungen können dann für jedes Voxel die beiden Unbekannten, die Konzentrationen der beiden Basismaterialien, bestimmt werden. Für viele Körpermaterialien führt diese Technik jedoch nicht zu zufriedenstellenden Ergebnissen, da die Zerlegung für alle im Körpermaterial enthaltenen Materialkomponenten schlecht vorhersehbar ist. So führt die Anwendung dieser Technik zur Bestimmung der Kontrastmittel-Konzentration in der Leber, die in der Regel auch größere Anteile an Fett enthält, zu einer schwer interpretierbaren Mischung aus den beiden Basismaterialien.

Weiterhin wird auf DE102006009222 A1 verwiesen, die eine 3-Material-Zerlegung eines untersuchten Gebietes mit Hilfe einer Dual-Energy-CT-Untersuchung ermöglicht. Hierbei wird das untersuchte Gebiet, vorzugsweise ein menschlicher oder tierischer Patient, in zwei unterschiedliche Gewebearten zerlegt und gleichzeitig das quantitative Vorkommen eines Kontrastmittels bestimmt.

Bei diesem letztgenannten Verfahren werden zwei Computertomographie-Aufnahmen des Körpermaterials mit einem Mehr-Energie-Computertomographen, insbesondere mit einem sog. Dual-Energy Computertomographen, bei zwei unterschiedlichen spektralen Verteilungen der Röntgenstrahlung aufgezeichnet. Die Aufzeichnung mit den beiden unterschiedlichen Röntgenenergien erfolgt hierbei vorzugsweise gleichzeitig. Aus den Messdaten der Computertomographie-Aufnahmen werden in bekannter Weise zwei Bilddatensätze rekonstruiert, die Röntgenschwächungswerte x enthalten. Unter Röntgenschwächungswerten können hierbei sowohl die Schwächungskoeffizienten $\mu$ als auch daraus abgeleitete Werte wie der CT-Wert verstanden werden.

Die Röntgenschwächungswerte x für jedes interessierende Voxel der beiden Bilddatensätze werden beim vorliegenden Verfahren in Röntgenschwächungswerte von drei Materialkomponenten zerlegt. Bei diesen drei Materialkomponenten handelt es sich um die beiden unterschiedlichen Materialkomponenten des Körpermaterials sowie um die Substanz, deren Konzentration bestimmt werden soll. Die beiden unterschiedlichen Materialkomponenten des Körpermaterials müssen dabei selbstverständlich keine chemisch reinen Materialien sein, sondern können auch Materialmischungen darstellen. Die Zerlegung der Röntgenschwächungswerte erfolgt beim vorliegenden Verfahren unter der Annahme, dass sich der Röntgenschwächungswert $x_M$ des Körpermaterials M ohne die Substanz aus den Röntgenschwächungswerten $x_{M1}$, $x_{M2}$ der ersten und zweiten Materialkomponente nach folgender Gleichung zusammensetzt:

$$x_M = f * x_{M1} + (1-f) * x_{M2},$$

wobei f einem Volumenanteil der ersten Materialkomponente im Körpermaterial entspricht. Auf Basis dieser Zerlegung wird dann die Konzentration der Substanz für jedes interessierende Voxel bestimmt. Dies ist möglich, da sich für jedes Voxel jeweils zwei Gleichungen entsprechend den beiden Bilddatensätzen mit insgesamt zwei Unbekannten ergeben, dem Volumenanteil f der ersten Materialkomponente sowie der Konzentration c der im Körpermaterial angereicherten Substanz.

Gemäß einer Ausgestaltung dieses Verfahrens wird die Konzentration der Substanz daher auch durch Lösung dieses Gleichungssystems der folgenden beiden Gleichungen bestimmt:

$$x_{E1} = c * x_{KM,E1} + f * x_{M1,E1} + (1-f) * x_{M2,E1}$$

$$x_{E2} = c * x_{KM,E2} + f * x_{M1,E2} + (1-f) * x_{M2,E2}$$

wobei $x_{E1/E2}$ den Röntgenschwächungswerten aus den beiden Bilddatensätzen bei den unterschiedlichen spektralen Energieverteilungen beziehungsweise Energien E1 und E2 der Röntgenstrahlung und c der Konzentration der Substanz im Körpermaterial entspricht. Die Röntgenschwächungswerte $x_{M1}$ und $x_{M2}$ bei den unterschiedlichen Röntgenenergien E1, E2 sind bekannt und können beispielsweise einer Tabelle entnommen werden. Das Gleiche gilt für den Röntgenschwächungswert $x_{KM}$ der zu bestimmenden Substanz. Dieser kann gegebenenfalls auch vorab durch eine getrennte Kalibriermessung, zum Beispiel unter Einsatz eines Wasserphantoms, bestimmt werden.

Das vorliegende Verfahren und die zugehörige Vorrichtung nutzen die Erkenntnis, dass in der Realität viele Materialien nur mit annähernd konstanter Dichte im menschlichen und tierischen Körper auftreten. Ausgehend von dieser Eigenschaft bedeutet dies, dass auch Mischungen zweier Materialien in einer CT-Aufnahme nicht mit beliebigen Röntgenschwächungswerten vertreten sind. Dies konnte für Lebergewebe experimentell belegt werden. Der CT-Wert von Lebergewebe nimmt mit zunehmendem Anteil an eingelagertem Fett linear ab. Außerdem ist bekannt, dass die Differenz zwischen den Röntgenschwächungswerten bei unterschiedlichen Röhrenspannungen des Computertomographen, d. h. bei unterschiedlichen Röntgenenergien, eine lineare Funktion des Fettgehaltes ist. Dieser Zusammenhang lässt sich auch auf andere Körpermaterialien übertragen und wird bei dem vorliegenden Verfahren und der zugehörigen Vorrichtung ausgenutzt.

Es lassen sich zwar durch alle oben genannte Verfahren zeitlich versetzt auch Untersuchungen mit mehreren Kontrastmitteln durchführen. Allerdings steigt dadurch die verwendete Strahlendosis proportional zur Anzahl der Untersuchungen an und der Zeitaufwand vergrößert sich entsprechend.

Es ist Aufgabe der Erfindung, ein verbessertes Verfahren zur Untersuchung mit und Darstellung von zwei unterschiedlichen in einem Patienten vorliegenden Kontrastmitteln zu finden, wobei eine Dosisreduktion erwünscht ist und hierfür die richtige Auswahl der beiden Kontrastmittel getroffen und eine optimierte Kontrastmittelkombination bestimmt werden soll.

Diese Aufgabe wird durch die Merkmale des unabhängigen Patentanspruchs gelöst. Vorteilhafte Weiterbildungen der Erfindung sind Gegenstand untergeordneter Ansprüche.

Die Erfinder haben erkannt, dass es möglich ist, eine CT-Untersuchung unter gleichzeitiger Anwendung von zwei Kontrastmitteln mit einer 3-Material-Zerlegungs-Methode durchzuführen, wobei es bei richtig ausgewählter Kombination der beiden Kontrastmittel möglich wird, einerseits ein reines Kontrastmittelbild des ersten Kontrastmittels zu erhalten und in einem zweiten Bild eine Darstellung zu erreichen, die einer Single-Energy-Aufnahme mit nur einem einzigen Kontrastmittel entspricht. Hierzu ist es zunächst notwendig, eine Kombination von zwei Kontrastmitteln auszuwählen, die ein erstes Kontrastmittel aufweist, welches eine möglichst hohe Enhancement-Steigung besitzt. Unter Enhancement-Steigung wird dabei das Verhältnis der HU-Wert-Anhebungen bei Zugabe von Kontrastmittel, also die Richtung beziehungsweise die Steigung einer Verbindungslinie unterschiedlicher Kontrastmittelkonzentrationen im HU-Werte-Diagramm über unterschiedliche Energiespektren angesehen. Unter Enhancement wird diese Verbindungslinie als Vektor betrachtet.

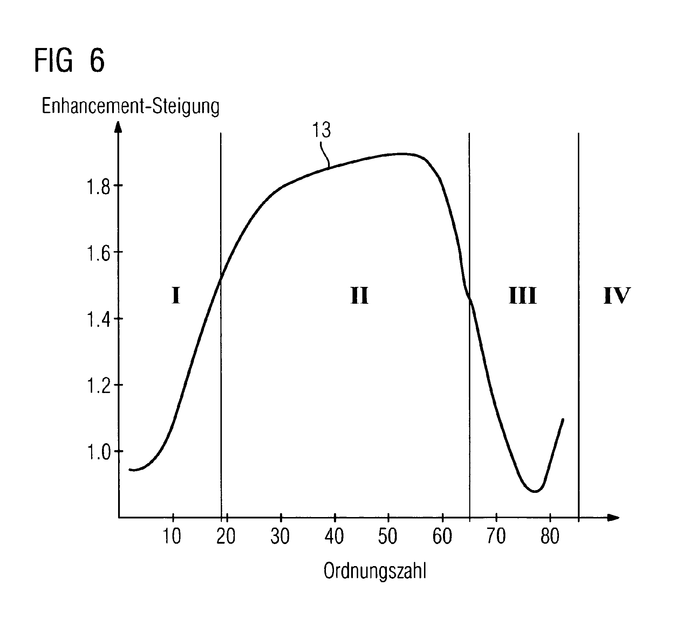

Diese Enhancement-Steigung muss nun so groß sein, dass eine Anreicherung einer Gewebeart zu einer, in einem HU-Werte-Diagramm aufgetragenen, Kontraststeigerung führt, die signifikant unterscheidbar oberhalb der in dem HU-Werte-Diagramm eingetragenen Verbindungslinie zwischen zwei untersuchten Gewebearten liegt. Da Körpermaterialien wie z.B. Fett oder Muskelgewebe im Wesentlichen aus leichten Atomen bis zu einer Ordnungszahl 8 (Sauerstoff) bestehen, liegt die Grundsteigung in der Regel nahe bei 1. Wie in der Figur 6 dargestellt, kann daher leicht ein Kontrastmittel verwendet werden, welches ein Element mit Ordnungszahl größer als 19 enthält. Weiterhin soll als zweites ein Kontrastmittel ausgewählt werden, welches eine Enhancement-Steigung aufweist, die der Grundsteigung zwischen den beiden untersuchten Gewebearten entspricht oder zumindest nicht signifikant und unterscheidbar von dieser Grundsteigung abweicht. Damit wird bei der Anwendung der 3-Material-Zerlegungs-Methode eine mit dem zweiten Kontrastmittel angereicherte erste oder zweite Gewebeart zunächst rechnerisch nicht unterscheidbar von einer Kombination der ersten und zweiten Gewebeart. Wird zusätzlich noch ein zweites Kontrastmittel gewählt, welches sich vorzugsweise an der im HU-Werte-Diagramm in Richtung höherer HU-Werte gelegenen Gewebeart anreichert, so wird zusätzlich für einen größeren Abstand der gemessenen HU-Werte, also für eine Kontrasterhöhung zwischen dem ersten und zweiten Gewebe, gesorgt. Zusätzlich kann auch differenziert werden zwischen Regionen die das zweite Gewebe enthalten und Kontrastmittel aufnehmen oder nicht.

Es wird darauf hingewiesen, dass der Begriff Gewebeart im Sinne der Erfindung keine Einschränkung bezüglich der betrachteten Materialien darstellen soll, beispielsweise kann als eine Gewebeart auch Luft verwendet werden.

Das Auswahlverfahren für die Kontrastmittel lässt sich auch auf die bekannte 2-Material-Zerlegung ausdehnen, da diese identisch ist zu einer 3-Material-Zerlegung, bei der Luft als erstes Material gewählt wird und z.B. Muskelgewebe als zweites Körpermaterial.

Werden nun mit Hilfe der in der DE 10 2006 009 222 beschriebenen Methode ein reines Kontrastmittelbild und ein virtuelles Nativbild erzeugt, so ist im Kontrastmittelbild ausschließlich die Information bezüglich des ersten Kontrastmittels enthalten, während im virtuellen Nativbild die Information bezüglich der beiden Gewebearten und des zweiten Kontrastmittels vorliegt. Beispielsweise lässt sich so einerseits eine Darstellung der Blutgefäße in einer Leber abbilden und gleichzeitig eine Darstellung der Leber mit bezüglich des Kontrastes verbesserter Auflösung zwischen normalem Lebergewebe und verfettetem Lebergewebe zeigen, beziehungsweise gesundem Lebergewebe und nicht Kontrastmittel aufnehmendem Tumor.

Ein wesentlicher Punkt der oben beschriebenen Erkenntnis liegt darin, dass es möglich ist, durch eine definierte und bezüglich ihrer Mengenverhältnisse vorbestimmte Kombination unterschiedlicher Kontrastmittelelemente oder Kontrastmittelkomponenten in einem Kontrastmittel ein gewünschtes Enhancement in einem HU-Werte-Diagramm zu erzeugen, welches auf der Verbindungslinie zweier Materialien im HU-Werte-Diagramm liegt. Beispielsweise kann dies dadurch geschehen, dass für das Kontrastmittel ein Kontrastmittelelement, z.B. aus der Gruppe der Lanthanide, ausgewählt wird, welches ein entsprechendes Enhancement beziehungsweise eine entsprechende Enhancement-Steigung erzeugt. Es können auch im molekularen Aufbau des Kontrastmittels mehrere Kontrastmittelelemente, vorzugsweise zumindest teilweise aus der Gruppe der Lanthanide, eingebaut werden, die in der Summe zum gewünschten Enhancement führen. Schließlich besteht auch die Möglichkeit als Kontrastmittel ein Gemisch unterschiedlicher Kontrastmittelkomponenten in einem vorbestimmten Verhältnis zueinander zu verwenden, die in der Summe das gewünschte Enhancement erzeugen, wobei darauf zu achten ist, dass diese einzelnen Kontrastmittelkomponenten die gleichen pharmakokinetischen Eigenschaften aufweisen, also sich im Körper identisch verhalten und somit keine Entmischungseffekte, zum Beispiel durch unterschiedliche Anlagerungen an Gewebestrukturen oder ähnliches, entstehen.

Entsprechend diesen Erkenntnissen schlagen die Erfinder ein Verfahren zur Auswahl zweier Kontrastmittel zur Verwendung in einer Dual-Energy-CT-Untersuchung eines Patienten gemäß Anspruch 1 vor.

Es ist hierbei anzumerken, dass der Signifikanzbereich der ermittelten Grundsteigung jeweils spezifisch für das verwendete CT-System ist und den Bereich darstellen soll, in dem eindeutig eine Abweichung der Enhancement-Steigung von der Grundsteigung detektierbar ist.

Erfindungsgemäß kann anstelle der beiden Materialien oder eines Materials eine Gewebeart treten. Im Folgenden steht der Begriff der Gewebeart sowohl für Material als auch Gewebeart.

Vorteilhaft ist es, wenn die Signifikanz zumindest in Bezug auf ein nachfolgend durchgeführtes Komponentenzerlegungsverfahren, die verwendete Untersuchungsdosis, die betrachteten Energiespektren und die Systemeigenschaften des CT-Systems bestimmt wird.

Als erste Gewebeart kann beispielsweise ein Drüsengewebe, vorzugsweise ein Lebergewebe, angesehen werden. Als zweite Gewebeart kann bevorzugt Fettgewebe angesehen werden. Weiterhin kann die Grundsteigung aus einer Vielzahl von CT-Leber-Untersuchungen mit unterschiedlichen Graden einer krankhaften Leberverfettung ermittelt werden.

Weiterhin kann in einer speziellen Variante der Erfindung als erstes Kontrastmittel ein jodhaltiges Kontrastmittel verwendet werden, während als zweites Kontrastmittel ein Kontrastmittel mit Anteilen aus der Gruppe der Lanthaniden genutzt wird. Hierbei ist es weiterhin möglich, im zweiten Kontrastmittel eine Kombination mindestens zweier Lanthaniden zu nutzen.

Bevorzugt wird als erstes Kontrastmittel ein Kontrastmittel gewählt, welches sich vorzugsweise im Blutkreislauf anreichert und als zweites Kontrastmittel ein Kontrastmittel gewählt, welches sich vorzugsweise in Zellen der ersten Gewebeart anreichert.

Des Weiteren wird ein Verfahren zur Erzeugung von CT-Aufnahmen eines Patienten, welcher zwei unterschiedliche Kontrastmittel aufweist, wobei das zweite Kontrastmittel ein Enhancement aufweist, dessen Steigung in einem HU-Werte-Diagramm verwendeter Strahlungsspektren der Steigung zwischen zwei unterschiedlichen Materialien oder Gewebearten (=Grundsteigung) in diesem HU-Werte-Diagramm entspricht, mit den folgenden Verfahrensschritten vorgeschlagen:

- scannen des Patienten mit einem CT-System, betrachtend mindestens zwei unterschiedliche Strahlungsspektren, und Rekonstruktion eines ersten Bilddatensatzes auf der Basis des ersten Strahlungsspektrums und eines zweiten Bilddatensatzes auf der Basis des zweiten Strahlungsspektrums,
- Erzeugung eines dritten Bilddatensatzes enthaltend ausschließlich die örtliche Konzentration des ersten Kontrastmittels und Erzeugung eines vierten Bilddatensatzes enthaltend die Gewebearten einschließlich der örtlichen Konzentration des zweiten Kontrastmittels durch Lösung von linearen Gleichungssystemen je Pixel/Voxel aus dem ersten und zweiten Bilddatensatz, die den Zusammenhang von Absorption und Anteilen / Konzentration der zwei Gewebearten und des ersten Kontrastmittels beschreiben,
- Ausgabe zumindest des dritten und/oder vierten Bilddatensatzes.

Hierbei kann die Auswahl der Kontrastmittel entsprechend dem zuvor beschriebenen Verfahren zur Auswahl zweier Kontrastmittel erfolgen.

Insbesondere kann je Pixel / Voxel folgendes lineares Gleichungssystem gelöst werden:

$$x_{E1} = c_{K1} * x_{K1,E1} + f * x_{G1,E1} + (1-f) * x_{G2,E1}$$

$$x_{E2} = c_{K1} * x_{K1,E2} + f * x_{G1,E2} + (1-f) * x_{G2,E2}$$

wobei die folgenden Variablen verwendet werden:

$f =$     Anteil der ersten Gewebeart G1,
$(1-f) =$     Anteil der zweiten Gewebeart G2,
$c_{K1} =$     Konzentration des ersten Kontrastmittels K1,
$x_{K1,E1} =$     bekannter Röntgenschwächungswert des ersten Kontrastmittels K1 bezogen auf das erste Energiespektrum E1,
$x_{K1,E2} =$     bekannter Röntgenschwächungswert des ersten Kontrastmittels K1 bezogen auf das zweite Energiespektrum E2,
$x_{G1,E1} =$     bekannter Röntgenschwächungswert der ersten Gewebeart G1 bezogen auf das erste Energiespektrum E1,
$x_{G1,E2} =$     bekannter Röntgenschwächungswert der ersten Gewebeart G1 bezogen auf das zweite Energiespektrum E2,
$x_{G2,E1} =$     bekannter Röntgenschwächungswert der zweiten Gewebeart G2 bezogen auf das erste Energiespektrum E1,
$x_{G2,E2} =$     bekannter Röntgenschwächungswert der zweiten Gewebeart G2 bezogen auf das zweite Energiespektrum E2.

Außerdem wird vorgeschlagen, dass zur Differenzierung des Absorptionsverhaltens der verwendeten Kontrastmittel bezüglich unterschiedlicher Energiespektren Messungen mit zwei unterschiedlichen Energiespektren der verwendeten Strahlung mit mindestens einem über mindestens die verwendeten Energiebereiche integrierenden Detektor durchgeführt werden.

Es können auch zur Differenzierung des Absorptionsverhaltens der verwendeten Kontrastmittel bezüglich unterschiedlicher Energiespektren Messungen mit einem Energiespektrum der verwendeten Strahlung und mindestens einem auf mindestens zwei unterschiedliche Energiebereiche selektivem Detektor durchgeführt werden.

Weiterhin wird eine Kombination zweier Kontrastmittel, vorgesehen zur Applikation bei einer CT-Untersuchung mit mindestens zwei unterschiedlichen diskret untersuchten Röntgenenergiespektren zur Beurteilung des Anteils einer ersten und einer zweiten Gewebeart, vorgeschlagen, wobei zwischen der ersten und zweiten Gewebeart in einem HU-Werte-Diagramm eine Verbindungslinie mit einer Grundsteigung gebildet wird, die Kombination bestehend aus:

- einem ersten Kontrastmittel, welches in Bezug auf die mindestens zwei verwendeten Energiespektren in einem HU-Werte-Diagramm eine Steigung aufweist, die signifikant höher ist als die Grundsteigung,
- einem zweiten Kontrastmittel, welches in Bezug auf die mindestens zwei verwendeten Energiespektren in dem HU-Werte-Diagramm eine Steigung aufweist, die im Signifikanzbereich der Grundsteigung liegt.

Hierbei kann die erste Gewebeart vorzugsweise ein Drüsengewebe, vorzugsweise ein Lebergewebe, sein und die zweite Gewebeart vorzugsweise ein erkranktes Drüsengewebe, vorzugsweise ein erkranktes Lebergewebe entsprechend dem Gewebe einer Fettleber, sein.

Weiterhin ist es vorteilhaft, wenn das erste Kontrastmittel sich vorzugsweise im Blutkreislauf anreichert und das zweite Kontrastmittel sich vorzugsweise in Zellen der ersten Gewebeart anreichert beziehungsweise sich an den darin befindlichen Gewebezellen anlagert.

Mindestens ein Kontrastmittel kann ein einziges Lanthanid enthalten, wodurch das gewünschte Enhancement erzeugt wird oder es kann mindestens ein Kontrastmittel aus einem Gemisch mehrerer Kontrastmittelkomponenten mit zumindest unterschiedlichen Lanthaniden bestehen, wobei die einzelnen Kontrastmittelkomponenten die gleichen pharmakokinetischen Eigenschaften und in der Summe das gewünschte Enhancement aufweisen sollten.

Andererseits kann auch mindestens ein Kontrastmittel einen molekularen Komplex aufweisen, welcher mit unterschiedlichen kontrastgebenden Elementen mit vorbestimmtem stöchiometrischen Verhältnis besetzt ist, so dass das gewünschte Enhancement erreicht wird.

Schließlich schlagen die Erfinder auch die Verwendung der erfindungsgemäßen Kombination zweier Kontrastmittel in dem eingangs beschriebenen Verfahren vor.

Im Folgenden wird die Erfindung anhand eines bevorzugten Ausführungsbeispieles mit Hilfe der Figuren näher beschrieben, wobei nur die zum Verständnis der Erfindung notwendigen Merkmale dargestellt sind. Dabei werden die folgenden Bezugszeichen verwendet: 1: CT-System; 2: erste Röntgenröhre; 3: erster Detektor; 4: zweite Röntgenröhre; 5: zweiter Detektor; 6: Gantrygehäuse; 7: Patient; 8: Patientenliege; 9: Systemachse, z-Achse; 10: Steuer- und Recheneinheit; 11: Kontrastmittelapplikator, 12: 3-Komponenten-Zerlegung; m: Grundsteigung zwischen Gewebe G1 und Gewebe G2; $m_1$: Enhancement-Steigung des ersten Kontrastmittels K1; $m_2$: Enhancement-Steigung des zweiten Kontrastmittels K2; A1: Durchführung eines CT-Scans mit einem ersten Energiespektrum; A2: Durchführung eines CT-Scans mit einem zweiten Energiespektrum; B1: CT-Bilddatensatz des CT-Scans mit dem ersten Energiespektrum; B2: CT-Bilddatensatz des CT-Scans mit dem zweiten Energiespektrum; $B_{K1}$: errechneter Bilddatensatz, darstellend die Verteilung des ersten Kontrastmittels K1; $B_{G1+G2+K2}$: errechneter Bilddatensatz, darstellend die Verteilung des zweiten Kontrastmittels K2 und der ersten und zweiten Gewebeart G1 und G2; G1: erste Gewebeart; G2: zweite Gewebeart; p: Röntgenabsorptionskoeffizient; $\mu$(80kVp): gemessene Absorptionskoeffizienten mit 80 kVp Röntgenstrahlung; $\mu$(140kVp): gemessene Absorptionskoeffizienten mit 140 kVp Röntgenstrahlung; I-IV: Ordnungszahlbereiche.

Es zeigen im Einzelnen:

FIG 1:  schematische Darstellung eines Computer-Tomographen;
FIG 2:  HU-Werte-Diagramm mit eingetragenen HU-Werten für ein erstes und ein zweites Gewebe;
FIG 3:  schematischer Verlauf der Absorptionskoeffizienten mit K-Kante über die Photonenenergie;
FIG 4:  HU-Werte-Diagramm für zwei Gewebearten einschließlich dem Enhancement einer erfindungsgemäßen Kombination zweier Kontrastmittel;
FIG 5:  schematische Darstellung der 3-Material-Zerlegung;
FIG 6:  schematische Darstellung der Enhancement-Steigung über die Kernladungszahl.

Entsprechend der Erfindung soll mit Hilfe der ComputerTomographie die Konzentration eines Kontrastmittels in Körpermaterialien, insbesondere in Lebergewebe, gemessen werden, wobei gleichzeitig ein anderes Kontrastmittel angewendet wird, bei dem nur die CT-Wert-Anhebung relativ zur Umgebung, nicht aber die exakte Konzentration von Interesse ist.

Die Lösung des Problems erfordert zwei spezielle Komponenten: Einerseits muss die Röntgenabsorption zumindest bezüglich zweier unterschiedlicher Energiespektren gemessen werden; vorzugsweise kann dies mit Hilfe eines Dual-Energy-CT-Scanners geschehen, der es erlaubt für mindestens einen axialen Schnitt durch den Patienten zwei unabhängige Bilder zu rekonstruieren, die mit verschiedenen effektiven Röntgenspektren erzeugt wurden. Dies lässt sich z.B. durch einen simultanen Scan mit zwei verschiedenen Röhrenspannungen auf einem CT mit zwei Röntgenröhren (=Dual Source CT) realisieren. Alternativ besteht zum Beispiel auch die Möglichkeit ein einfaches CT mit einem energieselektiven Detektor zu verwenden. Wichtig ist alleine, dass die unterschiedliche Absorptionswirkung auf zwei unterschiedliche Energiebereiche betrachtet werden kann. Nachfolgend soll der Begriff des Dual-Energy-CT allgemeingültig für ein energiespezifisches CT stehen.

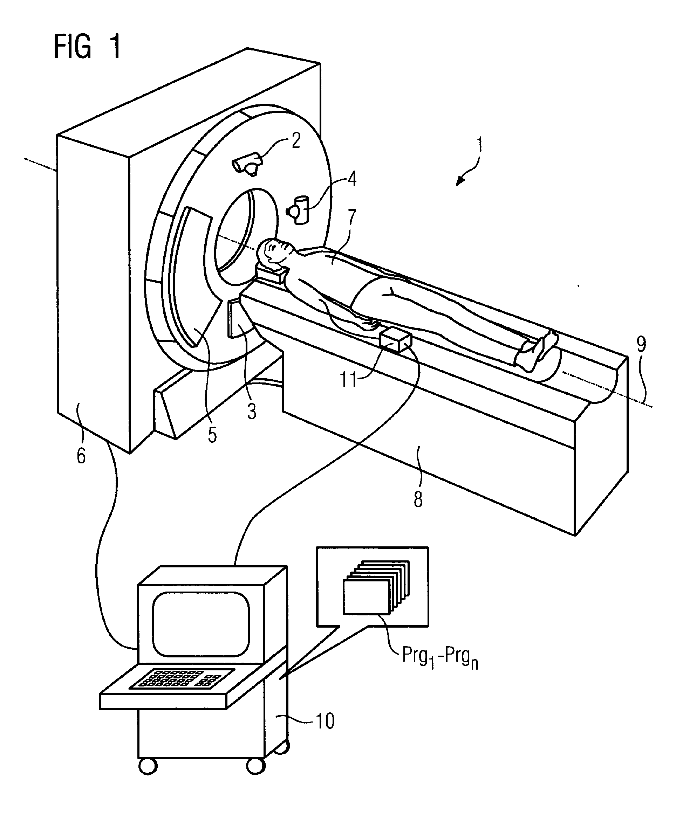

Ein Beispiel eines solchen CT-Systems 1 ist in der **Figur 1** dargestellt. Dieses CT-System 1 weist ein Gantrygehäuse 6 auf, in dem winkelversetzt zwei Röntgenröhren 2 und 4 mit gegenüberliegenden Detektorsystemen 3 und 5 angeordnet sind, die zur Abtastung des Patienten 7 um eine Systemachse 9 rotieren, während der Patient 7 durch die steuerbare Patientenliege entlang der Systemachse 9 durch den Messbereich des CT-Systems verschoben wird. In dem hier gezeigten Beispiel werden die beiden Röntgenröhren 2 und 4 mit unterschiedlichen Beschleunigungsspannungen betrieben, so dass die beiden verwendeten Röntgenspektren sich stark unterscheiden und auch unterschiedliche Absorptionswerte in den zugeordneten Detektoren bei der Durchstrahlung des Patienten liefern.

Zur Steuerung, Rekonstruktion und Durchführung des erfindungsgemäßen Verfahrens dient eine Steuer- und Recheneinheit 10, die in ihrem Speicher Computerprogramme $Prg_1$-$Prg_n$ beinhaltet, die im Betrieb die Steuerung und Rekonstruktion durchführen. Teil dieser Programme ist auch mindestens ein Programm Prg$_x$, welches ein erfindungsgemäßes Verfahren durchführt. Durch die Steuer- und Recheneinheit wird auch ein Kontrastmittelapplikator 11 gesteuert, mit dessen Hilfe die erfindungsgemäß ausgewählten Kontrastmittel appliziert werden können.

Zur Durchführung des erfindungsgemäßen Verfahrens werden speziell ausgewählte Kontrastmittel verwendet, die mit Hilfe von Dual-Energy-CT unterscheidbar sind. Als weitere Anforderung muss zumindest das zweite Kontrastmittel speziell auf die verwendeten effektiven Röntgenspektren und das betrachtete Gewebeumfeld bezüglich seines Absorptionsverhaltens ausgerichtet sein beziehungsweise ausgewählt werden.

Voraussetzung für die algorithmische Lösung des Problems ist eine an sich bekannte Dreimaterial-Zerlegung, wie in der DE 10 2006 009 222 beschrieben wird. Es lässt sich analog auch für eine konventionelle 2-Material-Zerlegung anwenden.

Nachfolgend wird das Verfahren speziell für eine Leberuntersuchung beschrieben, ohne jedoch die Allgemeingültigkeit beeinträchtigen zu sollen. Der Fachmann ist nach Kenntnis dieser speziellen Lösung ohne weiteres in der Lage die spezielle Lösung auf unterschiedliche andere Gewebekombinationen zu erweitern.

Betrachtet man das Lebergewebe in einem Patienten, so setzt sich dieses idealerweise nur aus Drüsengewebe, nachfolgend Gewebe genannt, und Fett mit jeweils konstanter Dichte zusammen. Auf Grund dieser Annahme gilt dann die folgende lineare Abhängigkeit des HU-Werts x in Abhängigkeit vom Fettgehalt f:

$$x = f \times x_{Fett} + (1-f) \times x_{Gewebe} .$$

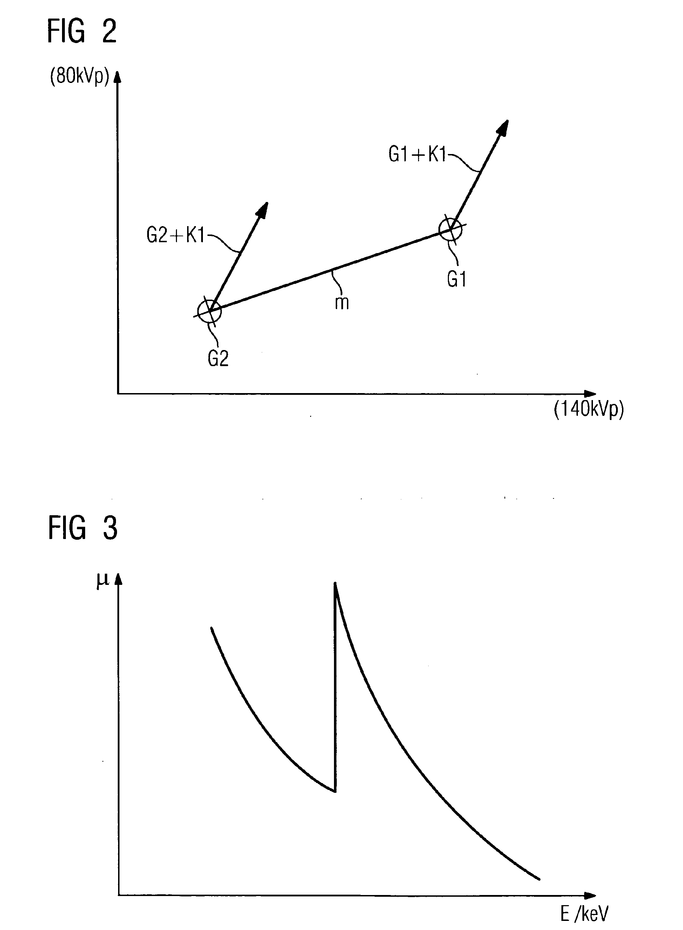

Dabei bezeichnen $x_{Fert}$ und $x_{Gewebe}$ die von der Röhrenspannung abhängigen HU-Werte der in den Indizes genannten reinen Materialien. Trägt man den ermittelten HU-Wert bei 80kVp Beschleunigungsspannung gegen den HU-Wert bei 140kVp Beschleunigungsspannung in einem HU-Werte-Diagramm, das in der **Figur 2** gezeigt ist, auf, so liegen die HU-Wert-Paare der HU-Werte für alle möglichen Mischungen aus Fett und Gewebe auf einer Geraden mit einer Steigung m, die der Grundsteigung im Sinne der Erfindung entspricht. In der Figur 2 ist das HU-Werte-Diagramm für die zwei Strahlungsspektren mit 80kVp auf der Ordinate und 140kVp auf der Abszisse dargestellt, wobei die Punkte G1 und G2 die HU-Werte-Paare von Gewebe und Fett darstellen.

Wird nun ein erstes Kontrastmittel K1, beispielsweise Jod, hinzugefügt, steigen die HU-Werte in beiden Spektren stark an. Dies wird in der Figur 2 durch die Pfeile G1+K1 und G2+K1 dargestellt. Diese dargestellten Pfeile entsprechen mit ihrer Steigung dem Enhancement des Kontrastmittels K1 bezogen auf die beiden verwendeten Energiespektren oder die betrachteten Energiebereiche. Da das Kontrastmittel selbst bei geringen Konzentrationen eine erhebliche Absorption hervorrufen, gilt näherungsweise, dass der HU-Wert linear mit der Konzentration c des beigemischten Kontrastmittels K1 zunimmt, wobei die Absorption pro molarer Konzentration des Kontrastmittels nicht vom organischen Material, hier der speziellen Fett/Gewebe-Mischung, abhängt. Der resultierende HU-Wert bezogen auf das 80kVp- beziehungsweise 140kVp-Spektrum ergibt sich damit zu:

$$x_{80/140} = f \times x_{Fett\,80/140} + (1-f) \times x_{Gewebe\,80/140} + c\,x_{Kontrastmittel\,80/140}$$

Mit Hilfe der eingangs erwähnten Dreikomponentenzerlegung lässt sich somit durch eine Messung der energiespezifischen HU-Werte für jeden Punkt im HU-Werte-Diagramm der Fettgehalt $f$ und die Kontrastmittelkonzentration c des einen Kontrastmittels K1 berechnen.

Dieses Verfahren funktioniert offensichtlich nur, wenn die Richtung der in Figur 1 eingezeichneten Vektoren, welche die CT-Wert-Anhebung durch das Kontrastmittel darstellt, also das Enhancement des Kontrastmittels, einen ausreichend großen Winkel mit der Verbindungsgeraden Fett/Gewebe aufspannt, da sonst das lineare Gleichungssystem mit dem die Komponenten-zerlegung berechnet wird, sehr empfindlich auf Rauschen reagiert. Im Extremfall, in dem das Enhancement beziehungsweise der Kontrastmittelvektor und die Verbindungsgerade parallel sind, ist das Gleichungssystem überhaupt nicht lösbar. Für existierende Kontrastmittel, die typischerweise schwere Atome bis hin zum Jod enthalten, ist dies in der Regel kein Problem. Speziell Jod-haltige Kontrastmittel sind für das Verfahren sogar optimal geeignet, da sie eine hohe Enhancement-Steigung aufweisen.

Um ein für das erfindungsgemäße Verfahren geeignetes Kontrastmittel K2 zu konstruieren, ist das folgende physikalische Hintergrundwissen nötig: Das Absorptionsspektrum von Jod wird durch den Photo-Effekt mit Auslösen eines Elektrons aus der K-Schale des Jods dominiert. Diese Absorption fällt für Photonen, die nicht genügend Energie für diesen Prozess haben (<33keV) stark ab, da dann nur die Elektronen der L-Schale zur Absorption beitragen. Diese "K-Kante" liegt für typische CT-Scanner unterhalb der verwendeten Photonen-Energie. Die Absorption hat schematisch einen Verlauf, wie er in der **Figur 3** gezeigt ist.

Für Elemente mit höherer Ordnungszahl bewegt sich die K-Kante von unten her in den vom Dual-Energy-CT-Scanner verwendeten Energie-Bereich. Dies führt dazu, dass bei konstanter Konzentration das Verhältnis der Absorption bei der niedrigeren Röhrenspannung relativ zur Absorption bei höherer Röhrenspannung abnimmt. Für geeignete hohe Ordnungszahlen kann der rekonstruierte HU-Wert bei der niedrigeren Röhrenspannung sogar kleiner werden als der HU-Wert bei der höheren Röhrenspannung. Zum Beispiel liegt dieser Übergang im Bereich der Lanthanide für derzeit existierende Scanner der Anmelder.

Es ist somit insbesondere möglich, ein Kontrastmittel K2 zu konstruieren, das in dem in Figur 2 dargestellten 80kVp/140kVp-HU-Werte-Diagramm einen Anhebungsvektor aufweist, der parallel zu der Verbindungslinie zwischen Fett und Gewebe liegt. Insbesondere kann eine Kombination aus zwei Kontrastmitteln K1 und K2 gefunden werden, wobei das erste Kontrastmittel K1 ein Enhancement aufweist, welches eine Steigung aufweist, die eindeutig und signifikant über der Grundsteigung zwischen zwei Gewebearten G1 und G2 liegt, und das zweite Kontrastmittel ein Enhancement aufweist, welches der vorgenannten Grundsteigung, zumindest im Bereich der Fehlertoleranz des CT-Systems, entspricht.

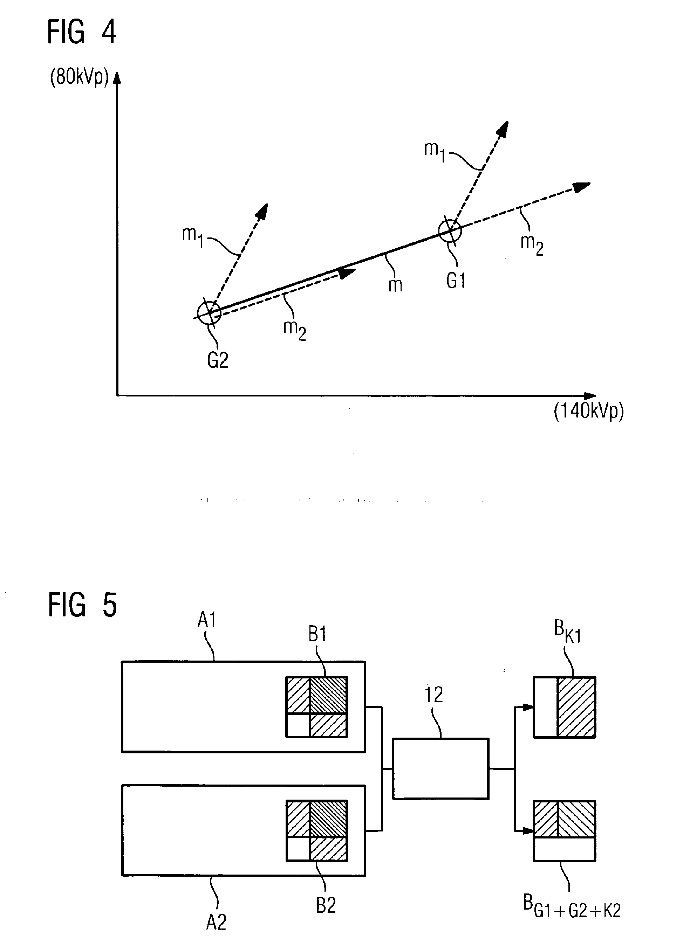

Eine solche Kombination ist schematisch in der **Figur 4** gezeigt. Hier sind die Punkte G1 und G2 der HU-Werte-Paare für die beiden Gewebearten Lebergewebe und Fettgewebe eingezeichnet. Die Verbindungsgerade G1-G2 zwischen diesen Punkten weist die Steigung m auf, wobei von jedem Punkt G1 und G2 Vektoren mit den Steigungen $m_1$ und $m_2$ ausgehen, die dem Enhancement der Kontrastmittel K1 und K2 entsprechen. Der Vektor der Steigung $m_2$ bei G2 ist zur besseren Erkennbarkeit etwas versetzt zur Verbindungslinie G1-G2 angeordnet. Das Kontrastmittel K2 wurde hier so gewählt beziehungsweise "designed", dass dessen Enhancement-Steigung $m_2$ der Grundsteigung m entspricht.

Wird unter diesen Bedingungen nun die geschilderte Dreimaterialzerlegung durchgeführt, so wird die Konzentration des Kontrastmittels K1 (z.B. mit Jod als Hauptkomponente) korrekt bestimmt, während das Kontrastmittel K2 (z.B. mit Erbium als Hauptkomponente) als Mischung aus Fett und Gewebe interpretiert wird, wobei der Faktor f im Ergebnis auch kleiner als Null sein kann.

Der diagnostische Nutzen des Verfahrens erschließt sich speziell, wenn ein virtuelles Nativbild berechnet wird. In diesem Bild wird das Kontrastmittel K2 zu einer entsprechenden HU-Wert-Anhebung führen. Man erhält somit einerseits ein reines Kontrastmittelbild für das Kontrastmittel K1 und ein "virtuelles Nativbild", welches wie ein traditionelles Single-Energy-CT-Bild unter Verwendung des Kontrastmittels K2 aussieht.

Dieses Verfahren ist insbesondere dann von Vorteil, wenn die Kontrastmittel unterschiedliche funktionale Aspekte des Körpergewebes darstellen, z.B. Vaskularisierung und Zellaktivität. Hierzu kann als Trägersubstanz/-molekül der beiden Kontrastmittel einerseits eine sich hauptsächlich im Blutkreislauf anreichernde Komponente für das erste Kontrastmittel K1 mit der höheren Enhancement-Steigung gewählt und als Trägersubstanz/-molekül für das zweite Kontrastmittel, eine andere sich hauptsächlich an erkranktem Gewebezellen, insbesondere Leberzellen, anreichernde Komponente gewählt werden. Gegenüber dem Verfahren der Registrierung/Subtraktion besteht der Vorteil, dass es keine Registrierungsartefakte gibt. Außerdem wird nur ein einziger CT-Scan benötigt und nicht zwei Scans, wie beim geschilderten alternativen Verfahren, so dass Zeit und Dosis eingespart wird.

Verkürzt lässt sich das erfindungsgemäße Verfahren entsprechend der **Figur 5** darstellen. Hier wird links zunächst die Aufnahme A1 und A2 zweier Bilddatensätze B1 und B2 unter Betrachtung unterschiedlicher Energiebereiche, zum Beispiel 50-80keV und 70-140keV, durchgeführt. Hierbei werden die erfindungsgemäß ausgewählten Kontrastmittel verwendet. Anschließend wird eine 3-Komponenten-Zerlegung im Verfahrensschritt 12 durchgeführt, beispielsweise eine 3-Komponenten-Zerlegung gemäß der in der Patentanmeldung mit Aktenzeichen DE 10 2006 009 222.8 beschriebenen Methode. Hieraus ergibt sich dann einerseits eine Darstellung $B_{K1}$ ausschließlich des ersten Kontrastmittels K1 und andererseits eine Darstellung $B_{G1+G2+K2}$ der beiden Gewebekomponenten G1 und G2 mit dem Kontrastmittel K2. Das erste Bild oder der erste Bilddatensatz entspricht damit einer Segmentierung des ersten Kontrastmittels K1, während das zweite Bild oder der zweite Bilddatensatz einer einfachen Aufnahme eines CTs mit einem Energiebereich unter Verwendung des zweiten Kontrastmittels K2 entspricht.

Schließlich ist in der **Figur 6** der Verlauf 13 der Enhancement-Steigung, also des Verhältnisses der HU-Wert-Anhebungen bei zwei beispielhaft verwendeten Röntgenspektren von 80 kVp - und 140 kVp - Röntgenstrahlung für ein ebenfalls beispielhaft verwendetes CT-System, gegenüber der Ordnungszahl des kontrasterzeugenden Elementes gezeigt. Das kontrasterzeugende Element wird hier beispielhaft in Wasser gelöst angenommen. Das Diagramm zeigt vier Bereiche I bis IV, wobei der Bereich I die Elemente beinhaltet, die wegen zu geringer Absorption und geringer Enhancement-Steigung als Kontrastmittel nicht geeignet sind. Im Bereich II befinden sich die Elemente die für das erste Kontrastmittel (bis Jod/frühe Lanthaniden) geeignet sind. Im Bereich III befinden sich die Elemente, die sich als zweites Kontrastmittel eignen, speziell die Lanthaniden. Während die im Bereich IV befindlichen Elemente als Kontrastmittel ungeeignet sind, da sie vorwiegend radioaktiv sind.

Es versteht sich, dass die vorstehend genannten Merkmale der Erfindung nicht nur in der jeweils angegebenen Kombination, sondern auch in anderen Kombinationen oder in Alleinstellung verwendbar sind, ohne den Rahmen der Erfindung zu verlassen.

**Patentansprüche**

1. verfahren zur Auswahl zweier Kontrastmittel zur Verwendung in einer Dual-Energy-CT-Untersuchung eines Patienten enthaltend die folgenden Verfahrensschritte:

1.1. Abtastung des Patienten (7) unter Betrachtung mindestens zweier unterschiedlicher Strahlungsspektren (E1, E2) und Rekonstruktion eines ersten Bilddatensatzes auf der Basis des ersten Strahlungsspektrums (E1) und eines zweiten Bilddatensatzes auf der Basis des zweiten Strahlungsspektrums (E2),
1.2. Bestimmung eines HU-Werte-Diagramms aus den zwei Bilddatensätzen,
1.3. Berechnung der Steigung ($m$)(=Grundsteigung) einer Verbindungslinie in dem HU-Werte-Diagramm der energiespezifischen HU-Werte zwischen einem ersten Material oder einer ersten Gewebeart (G1) und einem zweiten Material oder zweiten Gewebeart (G2),
1.4. Auswahl eines ersten Kontrastmittels (K1)
mit einer Enhancement-Steigung ($m_1$), welche höher ist als die ermittelte Grundsteigung ($m$),
1.5. Auswahl eines zweiten Kontrastmittels (K2) dessen Enhancement-Steigung ($m_2$) gleich der ermittelten Grundsteigung ($m$) ist.

2. Verfahren gemäß dem voranstehenden Patentanspruch 1, **dadurch gekennzeichnet, dass** als erste Gewebeart (G1) ein Drüsengewebe verwendet wird.

3. Verfahren gemäß einem der voranstehenden Patentansprüche 1 bis 2, **dadurch gekennzeichnet, dass** als erste Gewebeart (G1) ein Lebergewebe verwendet wird.

4. Verfahren gemäß einem der voranstehenden Patentansprüche 1 bis 3, **dadurch gekennzeichnet, dass** als zweite Gewebeart (G2) ein Fettgewebe verwendet wird.

5. Verfahren gemäß einem der voranstehenden Patentansprüche 1 bis 3, **dadurch gekennzeichnet, dass** als zweites Material (G2) Luft verwendet wird.

6. Verfahren gemäß einem der voranstehenden Patentansprüche 1 bis 4, **dadurch gekennzeichnet, dass** die Grundsteigung ($m$) aus einer Vielzahl von CT-Leber-Untersuchungen mit unterschiedlichen Graden einer krankhaften Leberverfettung ermittelt wird.

7. Verfahren gemäß einem der voranstehenden Patentansprüche 1 bis 6, **dadurch gekennzeichnet, dass** als erstes Kontrastmittel (K1) ein jodhaltiges Kontrastmittel ausgewählt wird.

8. Verfahren gemäß einem der voranstehenden Patentansprüche 1 bis 7, **dadurch gekennzeichnet, dass** als zweites Kontrastmittel (K2) ein Kontrastmittel mit Anteilen aus der Gruppe der Lanthaniden ausgewählt wird.

9. Verfahren gemäß dem voranstehenden Patentanspruch 8, **dadurch gekennzeichnet , dass** im zweiten Kontrastmittel (K2) eine Kombination mindestens zweier Lanthaniden ausgewählt wird.

10. Verfahren gemäß einem der voranstehenden Patentansprüche 1 bis 9, **dadurch gekennzeichnet, dass** als erstes Kontrastmittel (K1) ein Kontrastmittel gewählt wird, welches sich vorzugsweise im Blutkreislauf anreichert.

11. Verfahren gemäß einem der voranstehenden Patentansprüche 1 bis 10, **dadurch gekennzeichnet, dass** als zweites Kontrastmittel (K2) ein Kontrastmittel gewählt wir, welches sich vorzugsweise in Zellen der ersten Gewebeart anreichert.

**Claims**

1. Method for selecting two contrast agents to be used in a dual energy CT examination of a patient, comprising the following method steps:

1.1. scanning the patient (7) taking into account at least two different radiation spectra (E1, E2) and reconstructing a first image data record on the basis of the first radiation spectrum (E1) and a second image data record on the basis of the second radiation spectrum (E2),

1.2. determining an HU value diagram from the two image data records,

1.3. calculating the gradient (m) (= base gradient) of a connecting line between a first material or a first tissue type (G1) and a second material or second tissue type (G2) in the HU value diagram of the energy-specific HU values,

1.4. selecting a first contrast agent (K1) with an enhancement gradient (m1) which is greater than the determined base gradient (m),

1.5. selecting a second contrast agent (K2), the enhancement gradient of which (m2) is equal to the determined base gradient (m).

2. Method according to the preceding Patent Claim 1, **characterized in that** a glandular tissue is used as first tissue type (G1).

3. Method according to one of the preceding Patent Claims 1 to 2, **characterized in that** a liver tissue is used as first tissue type (G1).

4. Method according to one of the preceding Patent Claims 1 to 3, **characterized in that** a fatty tissue is used as second tissue type (G2).

5. Method according to one of the preceding Patent Claims 1 to 3, **characterized in that** air is used as second material (G2).

6. Method according to one of the preceding Patent Claims 1 to 4, **characterized in that** the base gradient (m) is determined from a multiplicity of CT liver examinations with different degrees of pathological fatty metamorphosis of liver.

7. Method according to one of the preceding Patent Claims 1 to 6, **characterized in that** an iodine-containing contrast agent is selected as first contrast agent (K1).

8. Method according to one of the preceding Patent Claims 1 to 7, **characterized in that** a contrast agent with components from the group of the lanthanides is selected as second contrast agent (K2).

9. Method according to the preceding Patent Claim 8, **characterized in that** a combination of at least two lanthanides is selected in the second contrast agent (K2).

10. Method according to one of the preceding Patent Claims 1 to 9, **characterized in that** a contrast agent which preferably accumulates in the cardiovascular system is selected as first contrast agent (K1).

11. Method according to one of the preceding Patent Claims 1 to 10, **characterized in that** a contrast agent which preferably accumulates in cells of the first tissue type is selected as second contrast agent (K2).

**Revendications**

1. Procédé de sélection de deux agents de contraste à utiliser lors d'un examen par CT à énergie dual d'un patient, comprenant les stades de procédé suivants :

1.1. balayage du patient ( 7 ), en considérant au moins deux spectres ( E1, E2 ) de rayonnement différents, et reconstruction d'un premier jeu de données d'image sur la base du premier spectre ( E1 ) de rayonnement et d'un deuxième jeu de données d'image sur la base du deuxième spectre ( E2 ) de rayonnement,

1.2. détermination d'un graphique de valeurs HU à partir des deux jeux de données d'image,

1.3. calcul de la pente ( $m$ ) ( = pente de base ) d'une ligne de liaison dans le graphique de valeurs HU des valeurs HU spécifiques à l'énergie entre un premier matériau ou un premier type ( G1 ) de tissu et un deuxième matériau ou un deuxième type ( G2 ) de tissu,

1.4. sélection d'un premier agent ( K1 ) de contraste ayant une pente ( $m_1$ ) d'enhancement, qui est plus grande que la pente ( $m$ ) de base déterminée,

1.5. sélection d'un deuxième agent ( K2 ) de contraste, dont la pente ( $m_2$ ) d'enhancement est égale à la pente ( $m$ ) de base déterminée.

2. Procédé suivant la revendication précédente 1, **caractérisé en ce qu'**on utilise un tissu ganglionnaire comme premier type ( G1 ) de tissu.

3. Procédé suivant l'une des revendications précédentes 1 à 2, **caractérisé en ce qu'**on utilise un tissu hépatique comme type ( G1 ) de tissu.

4. Procédé suivant l'une des revendications précédentes 1 à 3, **caractérisé en ce qu'**on utilise un tissu adipeux comme deuxième type ( G2 ) de tissu.

5. Procédé suivant l'une des revendications précédentes 1 à 3, **caractérisé en ce qu'**on utilise de l'air comme deuxième matière ( G2 ).

6. Procédé suivant l'une des revendications précédentes 1 à 4, **caractérisé en ce qu'**on détermine la pente ( $m$ ) de base à partir d'une pluralité d'examens hépatiques CT ayant des degrés différents d'adipose hépatique maladive.

7. Procédé suivant l'une des revendications précédentes 1 à 6, **caractérisé en ce qu'**on choisit un agent de contraste iodé comme premier agent ( K1 ) de contraste.

8. Procédé suivant l'une des revendications précédentes 1 à 7, **caractérisé en ce qu'**on choisit comme deuxième agent ( K2 ) de contraste un agent de contraste ayant des parties du groupe des lanthanides.

9. Procédé suivant la revendication précédente 8, **caractérisé en ce qu'**on choisit dans le deuxième agent ( K2 ) de contraste une combinaison d'au moins deux lanthanides.

10. Procédé suivant l'une des revendications précédentes 1 à 9, **caractérisé en ce qu'**on choisit, comme premier agent ( K1 ) de contraste, un agent de contraste, qui s'enrichit, de préférence, dans la circulation sanguine.

11. Procédé suivant l'une des revendications précédentes 1 à 10, **caractérisé en ce qu'**on choisit, comme deuxième agent ( K2 ) de contraste, un agent de contraste, qui s'enrichit, de préférence, dans des cellules du premier type de tissu.

FIG 1

## FIG 2

## FIG 3

## FIG 4

## FIG 5

## FIG 6

Enhancement-Steigung

Ordnungszahl

**IN DER BESCHREIBUNG AUFGEFÜHRTE DOKUMENTE**

**In der Beschreibung aufgeführte Patentdokumente**

- DE 102006009222 A1
- DE 102006009222